# EUROPÄISCHE PATENTSCHRIFT

(11) **EP 1 011 674 B1**
(45) Veröffentlichungstag und Bekanntmachung des Hinweises auf die Patenterteilung: **20.03.2002**
(21) Anmeldenummer: 98946364.1
(22) Anmeldetag: 18.08.1998
(51) Int. Cl.: A61K 31/46, A61K 9/70

(54) **TRANSDERMALES THERAPEUTISCHES SYSTEM MIT DEM WIRKSTOFF SCOPOLAMINBASE**
TRANSDERMAL THERAPEUTIC SYSTEM CONTAINING THE ACTIVE SUBSTANCE SCOPOLAMINE BASE
SYSTEME THERAPEUTIQUE TRANSDERMIQUE CONTENANT LE PRINCIPE ACTIF SCOPOLAMINE BASE

(30) Priorität: 04.09.1997 DE 19738643
(43) Veröffentlichungstag der Anmeldung: 28.06.2000
(73) Patentinhaber: LTS Lohmann Therapie-Systeme AG, 56626 Andernach (DE)
(72) Erfinder: MÜLLER, Walter, D-56564 Neuwied (DE)
(74) Vertreter: Flaccus, Rolf-Dieter, Dr.
(86) Internationale Anmeldenummer: EP9805224
(87) Internationale Veröffentlichungsnummer: WO9911265

(56) Entgegenhaltungen:
- EP-A- 0 180 377
- EP-A- 0 671 176
- WO-A-97/20550
- DE-A- 4 438 989
- US-A- 3 797 494
- US-A- 3 903 880
- US-A- 4 832 953

## Beschreibung

Die Erfindung betrifft ein transdermales therapeutisches System mit dem Wirkstoff Scopolaminbase, umfassend eine flexible, wirkstoffundurchlässige Rückschicht, eine wirkstoffhaltige Reservoirschicht, eine Steuermembran, eine Haftklebschicht zur Befestigung des Systems auf der Haut, sowie eine vor Applikation zu entfernende, ebenfalls wirkstoffundurchlässige Schutzfolie.

Scopolamin ist ein bekannter Wirkstoff, der sich mit Hilfe eines Pflasters für transdermale Applikation mit systemischer Wirkung eignet. Es handelt sich dabei um ein sogenanntes Antiemetikum, das bevorzugt zur Vermeidung von Übelkeit und Erbrechen, beispielsweise als Folge von wiederholten passiven Veränderungen des Gleichgewichts auf Reisen, eingesetzt wird.

Der therapeutische Vorteil einer transdermalen Verabreichung besteht darin, daß die Wirkstoffzufuhr langsam und kontinuierlich unter Steuerung durch das transdermale System erfolgt. Es ist dadurch möglich, das vergleichsweise enge therapeutische Fenster für Scopolamin zuverlässig zu treffen und einerseits dadurch einen therapeutisch effektiven Plasmaspiegel einzustellen, ohne andererseits die mit einer Überdosis eintretenden Nebenwirkungen, wie z.B. Mundtrockenheit, Übelkeit und Blendempfindlichkeit der Augen, befürchten zu müssen.

Die US 3,797,494 beschreibt ein bekanntes transdermales therapeutisches System, daß zur Verabreichung von Scopolamin mit systemischer Wirkung verwendet wird. Es besteht im wesentlichen aus einer Rückschicht, einem Wirkstoffreservoir, einer mikroporösen Membran, einer ebenfalls wirkstoffhaltigen Hautklebeschicht und einer vor Gebrauch zu entfernenden Schutzfolie. Das Reservoir und die Hautklebeschicht enthalten eine Mischung von Polyisobutylenen mit verschiedenen Molekulargewichten und Mineralöl. In dieser Mischung ist der Wirkstoff als viskose Flüssigkeit dispergiert.

Ein transdermales System, dessen wirkstoffhaltigen Bestandteile auf dieser Basis aufgebaut sind, zeigt jedoch gravierende Nachteile. Unter bestimmten Bedingungen kann es nämlich zu einer spontanen Kristallisation des Wirkstoffs kommen, wodurch die Bioverfügbarkeit des Wirkstoffes im Pflaster negativ beeinflußt und gegebenenfalls völlig aufgehoben wird.
Die US 4,832,953 beschreibt ausführlich die Ursachen und Folgen derartiger Instabilität. Es wird ein Verfahren beschrieben, wie durch nachträgliche Hitzebehandlung des schon verpackten Pflasters die Kristallisation verhindert werden kann. Dabei bleibt aber das Ergebnis unsicher und ist nicht kontrollierbar. Entsprechend den Angaben in diesem Dokument ist vor allem Scopolaminhydrat an der unerwünschten Kristallisation beteiligt.

Es ist von größtem Nachteil, wenn unter nicht definierbaren Bedingungen in einer transdermalen Arzneiform enthaltener Wirkstoff zu einer nicht vorhersagbaren Zeit nach Herstellung seine Aggregatform unter negativer Beeinflussung der Bioverfügbarkeit ändert.

Die DE-OS 44 38 989 beschreibt Systeme auf der Basis von Polyacrylatklebern mit Scopolaminbase. Bei diesen wird die Rekristallisation des Wirkstoffs dadurch verhindert, daß der Wirkstoff in vollständiger Lösung mit einer Konzentration unterhalb der Sättigungsgrenze vorliegt. Nachteilig ist hierbei, daß der Wirkstoff in vivo mit einer Kinetik 1. Ordnung abgegeben wird und infolgedessen bei einer Wirkstoffbeladung zwischen 1,5 und 2 mg die Permeationsrate über die Applikationszeit von 3 Tagen abfällt und infolgedessen nicht konstant ist. Erst bei einer höheren Wirkstoffbeladung wäre die Wirkstoffabgabe über die Applikationszeit ausreichend konstant, erfolgt aber immer noch nicht nach einer Kinetik 0. Ordnung. Auch eine Verwendung von Membranen kann dabei das Problem nicht lösen, weil zwar die Abgaberate, nicht jedoch die Art der Kinetik beeinflußt wird.

Ausgehend von diesem Stand der Technik liegt der Erfindung die Aufgabe zugrunde, ein transdermales therapeutisches System mit dem Wirkstoff Scopolaminbase der im Oberbegriff von Anspruch 1 genannten Art anzugeben, welches die vorgenannten Nachteile von Pflastern nach dem Stand der Technik vermeidet und insbesondere
a. sicher eine Rekristallisation des Wirkstoffs vermeidet,
b. mit einer Wirkstoffbeladung von nicht mehr als 2 mg eine zufriedenstellende Abgaberate ergibt,
c. nach Einstellung eines Permeations-Gleichgewichts von Wirkstoff durch die Haut eine konstante Abgaberate über den vorgesehenen Applikationszeitraum verwirklicht und konstante Plasmaspiegel liefert.

Überraschenderweise gelingt die Lösung der Aufgabe mit der Erfindung bei dem eingangs erwähnten transdermalen therapeutischen System mit dem Wirkstoff Scopolaminbase dadurch,
- daß die Reservoirschicht sowie die Haftklebschicht aus einem selbstklebenden aminresistenten Silikonpolymer als Basispolymer bestehen,
- daß die Steuermembran aus einem Ethylenvinylacetat-Copolymer besteht, und
- daß die Scopolaminbase zumindest in der Reservoirschicht zu einem Teil in kristalliner Form vorliegt.

Um den Wirkstoff über die bei solchen Pflastern übliche Tragzeit von beispielsweise 3 Tagen konstant abgeben zu können, darf die thermodynamische Aktivität des Wirkstoffs im Reservoir über die Tragzeit nicht wesentlich abnehmen. Um dies zu erreichen, muß die Konzentration des gelösten Anteils an Scopolaminbase im Reservoir bzw. in der Klebschicht konstant bleiben. Dies gelingt nur, wenn ein Teil des Wirkstoffs zunächst ungelöst vorliegt und sich erst während der Applikationszeit stetig nachlöst. Überraschenderweise hat sich herausgestellt, daß Silikonkleber hierfür das bestgeeignete Basispolymer darstellen, das über ein ausreichendes, jedoch nicht zu hohes Lösevermögen für Scopolaminbase verfügt. Aminresistente Silikonkleber zeichnen sich dadurch aus, daß sie über keine freien Si-O-H Gruppen verfügen. Die in normalen Silikonklebern vorhandenen Si-O-H Gruppen werden dazu in einem speziellen Herstellschritt umgewandelt in Si-O-CH₃ Gruppen. Derartige Kleber sind in der EP-A 0 180 377 beschrieben.
Darüber hinaus sind Silikonkleber in unpolaren Lösemitteln, wie z.B. n-Heptan, gut löslich, die andererseits aber über ein äußerst geringes Lösevermögen für Scopolaminbase verfügen. Es besteht daher bei Verwendung von Silikonkleber als Basispolymer die Möglichkeit, den Wirkstoff in eine Lösung der Silikonkleber einzuarbeiten, ohne daß der Wirkstoff komplett gelöst wird. Während der Entfernung der Lösemittel dürfen dabei nur Temperaturen angewendet werden, die unterhalb der Schmelztemperatur von Scopolaminbase liegen, weil ansonsten der Wirkstoff in Form einer unterkühlten Schmelze in dem Kleber dispergiert werden würde. Tröpfchen aus unterkühlter Schmelze könnten dann zu nicht vorhersehbarer Zeit rekristallisieren.

Weil jedoch der Wirkstoff nach der Erfindung zum Teil zumindest in der Reservoirschicht in kristalliner Form vorliegt, bleibt seine thermodynamische Aktivität im Polymer nach Einstellung eines Gleichgewichts bei der Wirkstoffabgabe aus der Reservoirschicht in die Klebschicht und beim Nachlösen des kristallinen Anteils annähernd konstant. Die thermodynamische Aktivität ist dabei aufgrund des hohen Diffusionskoeffizienten im Silikonkleber gleich bzw. nahe dem Maximum. Dies bedeutet, daß die Permeationsrate unter in vivo-Bedingungen zu hoch wäre, um bei einer Pflastergröße von 2 bis 3 cm² Nebenwirkungen aufgrund zu hoher Plasmaspiegel vermeiden zu können. Deshalb ist zur Kontrolle der Permeationsrate die Anordnung einer Steuermembran zwischen Reservoirschicht und Klebschicht unabdingbar erforderlich. Als geeignet haben sich Steuermembranen auf der Basis von Ethylenvinylacetat erwiesen. Die Durchlässigkeit dieser Membranen für den Wirkstoff lassen sich nach Maßgabe von Dicke und dem Vinylacetatgehalt der Membran einstellen. Als geeignet haben sich im Sinne der Erfindung Membranen erwiesen, die eine Dicke von 30 bis 100 um und einen Vinylacetatgehalt von 4,5 bis 19 % aufweisen.
Vorteilhaft ist auch der Einsatz von Substanzen, welche die Permeationsrate durch menschliche Haut erhöhen. Solche Permeationsverstärker reduzieren die Barrierewirkung der menschlichen Haut und damit auch deren individuellen Einfluß auf die Wirkstoffaufnahme. Infolgedessen können Permeationsverstärker die Kontrolle der Wirkstoffaufnahme durch das Pflastersystem wesentlich erhöhen und die von Patient zu Patient unterschiedlichen Schwankungen der Permeationsrate sowie die dadurch resultierenden Schwankungen der Plasmaspiegel wesentlich reduzieren. Bevorzugt werden Fettsäuren oder Fettalkohole eingesetzt. Als besonders geeignet haben sich dabei Ölsäure und Oleylaikohol erwiesen, welche in den eingesetzten Konzentrationen nicht zu Hautreizungen führen und kompatibel mit Silikonklebern sind. Ein zusätzlicher Vorteil dieser Substanzen ergibt sich daraus, daß sie die sehr geringe Löslichkeit von Scopolaminbase in den Silikonklebern erhöhen.
Dem Silikonkleber können auch andere, die Löslichkeit des Wirkstoff erhöhende Substanzen, wie beispielsweise Polymere, zugesetzt werden. Andere Zusätze, wie z.B. Kieselgele mit einer hohen spezifischen Oberfläche, können verwendet werden, um die physikalischen Eigenschaften der Kleberstriche, beispielsweise deren Kohäsion, zu verbessern.

Im folgenden werden Beispiele für die Herstellung eines erfindungsgemäßen transdermalen therapeutischen Systems beschrieben.

### Beispiel 1:

### A Herstellung der Hautkontaktschicht

In einem Masseverhältnis von ca. 1 : 44,1 (g/g) werden 9,5 g Scopolaminbase zu 419,3 g eines aminresistenten Silikonklebers mit einem Feststoffgehalt von 80% (Lösemittel n-Heptan) gegeben und die Lösung durch Rühren homogenisiert. Die Lösung wird mit einer Rakel auf eine silikonisierte Polyesterfolie beschichtet und für 30 min. bei 50 °C das Lösemittel entfernt. Das Beschichtungsgewicht des getrockneten Films wird mit 30 g/m² eingestellt. Der getrocknete Film wird durch eine EVA-Membran mit einer Dicke von 50 µm und einem Vinylacetatgehalt von 9% abgedeckt.

### B Herstellung der Reservoirschicht

In einem Masseverhältnis von ca. 1:13,66 (g/g) werden 25,3 g Scopolaminbase zu 345,5 g eines aminresistenten Silikonklebers mit einem Feststoffgehalt von 80% (Lösemittel n-Heptan) gegeben und die Lösung durch Rühren homogenisiert. Die Lösung wird mit einer Rakel auf eine silikonisierte Polyesterfolie beschichtet und für 30 min. bei 50 °C das Lösungsmittel entfernt. Das Beschichtungsgewicht des getrockneten Films wird mit 60 g/m² eingestellt. Der getrocknete Film wird nun mit einer 25 µm dicken Polyesterfolie abgedeckt.

### C Herstellung des Gesamtlaminats

Von der unter B hergestellten Reservoirschicht wird die silikonisierte Polyesterfolie entfernt, die Reservoirschicht auf die EVA-Membran der unter A hergestellten Hautkontaktschicht kaschiert. Aus dem Gesamtlaminat werden 2,5 cm² große Pflaster ausgestanzt.

### Beispiel 2:

Die Herstellung erfolgt analog Beispiel 1, allerdings werden den Silikonkleberlösungen 1% (g/g) Ölsäure (bezogen auf den Feststoffgehalt) zugesetzt.

### Beispiel 3:

Die Herstellung erfolgt analog Beispiel 1, allerdings werden den Silikonkleberlösungen 1% (g/g) Oleylalkohol (bezogen auf den Feststoffgehalt) zugesetzt.

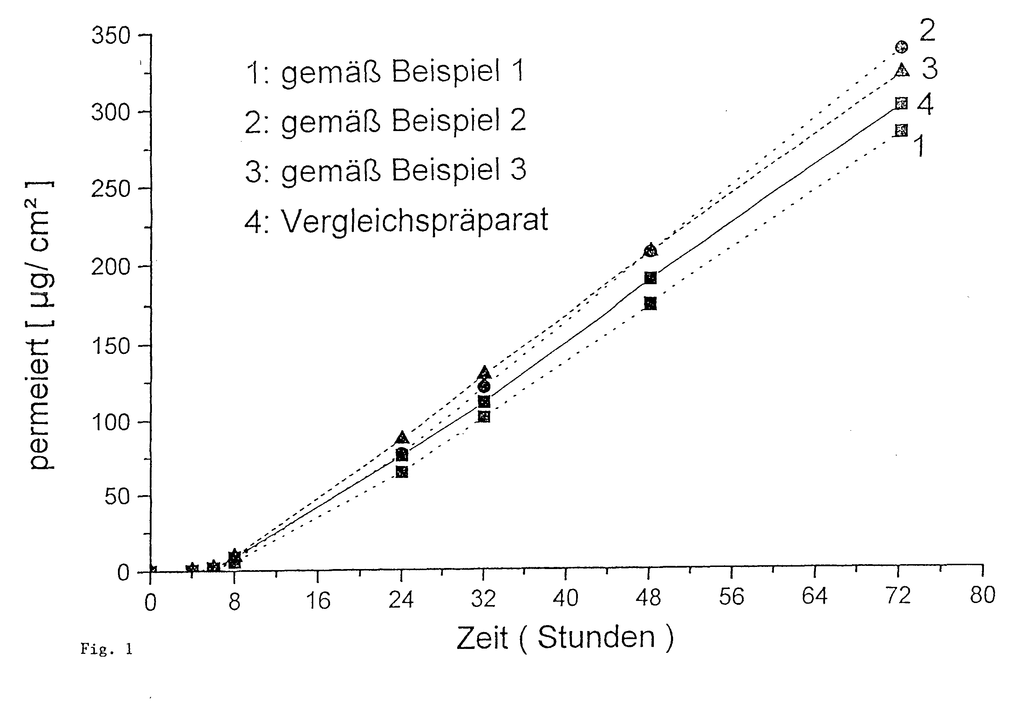

Die Ergebnisse von vergleichenden Permeationsversuchen zwischen einem handelsüblichen Vergleichspräparat mit drei Systemen nach der Erfindung werden in einem Diagramm gemäß Figur 1 graphisch dargestellt. Jede Kurve stellt den Mittelwert aus 3 Messungen dar. Die Systeme wurden gemäß den Beispielen 1 bis 3 hergestellt. Für die Bestimmung wurden sogenannten Franzdiffusionszellen unter Verwendung von menschlichen Epidermen benutzt.
Die Ergebnisse zeigen, daß die Permeationsprofile der Systeme gemäß der Erfindung mit dem Vergleichspräparat annähernd identisch sind. Dies beweist, daß die Systeme entsprechend der Erfindung bezüglich ihrer Permeationsraten die gleichen Eigenschaften aufweisen wie das Vergleichspräparat, ohne dessen Nachteile bezüglich der Rekristallisationsgefahr aufzuweisen.

## Patentansprüche

1. Transdermales therapeutisches System mit dem Wirkstoff Scopolaminbase, umfassend eine flexible, wirkstoffundurchlässige Rückschicht, eine wirkstoffhaltige Reservoirschicht, eine Steuermembran, eine Haftklebeschicht zur Befestigung des Systems auf der Haut, sowie eine vor Applikation zu entfernende, ebenfalls wirkstoffundurchlässige Schutzfolie, **dadurch gekennzeichnet,**
- **daß** die Reservoirschicht sowie die Haftklebschicht aus einem selbstklebenden aminresistenten Silikonpolymer als Basispolymer bestehen,
- **daß** die Steuermembran aus einem Ethylenvinylacetat-Copolymer besteht, und
- **daß** die Scopolaminbase zu einem Teil zumindest in der Reservoirschicht in kristalliner Form vorliegt.

2. Transdermales therapeutisches System nach Anspruch 1, **dadurch gekennzeichnet, daß** die Steuermembran einen Gehalt an Vinylacetat zwischen 4,5 und 19% aufweist und eine Dicke zwischen 30 und 100 µm besitzt.

3. Transdermales therapeutisches System nach Anspruch 1 oder 2, **dadurch gekennzeichnet, daß** die Reservoirschicht einen Gehalt an Scopolaminbase zwischen 1,0 und 2,5 mg (g/g), vorzugsweise 1,5 - 2,0 mg, aufweist.

4. Transdermales therapeutisches System nach Anspruch 1 oder 2, **dadurch gekennzeichnet, daß** die Haftklebschicht einen Gehalt an Scopolaminbase zwischen 0,2 und 1,0 mg (g/g), vorzugsweise 0,2 bis 0,7 mg, aufweist.

5. Transdermales therapeutisches System nach einem oder mehreren der Ansprüche 1 bis 4, **dadurch gekennzeichnet, daß** sowohl die Reservoir- als auch die Haftklebschicht eine die Permeationsrate durch menschliche Haut begünstigende Substanz, vorzugsweise eine Fettsäure oder Fettalkohol, enthält.

6. Transdermales therapeutisches System nach Anspruch 5, **dadurch gekennzeichnet, daß** der Permeationsverstärker Ölsäure ist.

7. Transdermales therapeutisches System nach Anspruch 5, **dadurch gekennzeichnet, daß** der Permeationsverstärker Oleylalkohol ist.

8. Verfahren zur Herstellung eines scopolaminhaltigen Pflasters entsprechend den Ansprüchen 1 bis 7, bei dem das Herstellverfahren für die wirkstoffhaltigen Schichten folgende Schritte umfaßt:
a) Suspendieren der Scopolaminbase in einer Lösung des aminresistenten Silikonklebers
b) Beschichten einer Suspension auf eine geeignete Folie
c) Entfernen der Lösemittel bei Temperaturen unterhalb der Schmelztemperatur der Scopolaminbase.

## Claims

1. Transdermal therapeutic system containing the active substance scopolamine base and comprising a flexible, active substance-impermeable backing layer, an active substance-containing reservoir layer, a control membrane, a pressure-sensitive-; adhesive, layer for attaching:.the system onto the skin, as well as a protective film or sheet which is likewise active substance-impermeable and is to be removed prior to application, **characterized in that**
- the reservoir layer as well as the pressure-sensitive adhesive layer are made up of a self-adhesive aminoresistant silicone polymer as base polymer,
- the control membrane is made up of an ethylene-vinyl acetate copolymer, and that
- part of the scopolamine base is present, at least in the reservoir layer, in crystalline form.

2. Transdermal therapeutic system according to claim 1, **characterized in that** the control membrane has a vinyl acetate content of between 4.5 and 19%, and a thickness between 30 and 100 µm.

3. Transdermal therapeutic system according to claim 1 or 2, **characterized in that** the reservoir layer has a content of scopolamine base of between 1.0 and 2.5 mg (w/w), preferably 1.5 to 2.0 mg.

4. Transdermal therapeutic system according to claim 1 or 2, **characterized in that** the pressure-sensitive adhesive layer has a content of scopolamine base of between 0.2 and 1.0 mg (w/w), preferably 0.2 to 0.7 mg.

5. Transdermal therapeutic system according to any one or several of claims 1 to 4, **characterized in that** both the reservoir layer and the pressure-sensitive adhesive layer contain a substance enhancing the permeation rate through human skin, preferably a fatty acid or fatty alcohol.

6. Transdermal therapeutic system according to claim 5, .**characterized in that** the permeation enhancer is oleic acid.

7. Transdermal therapeutic system according to claim 5, **characterized in that** the permeation enhancer is oleyl alcohol.

8. Process for the production of a scopolamine-containing patch according to claims 1 to 7, wherein the production process for the active substance-containing layers comprises the following steps:
a) suspending the scopolamine base in a solution of the aminoresistant silicone adhesive
b) coating a suspension onto a suitable film or sheet
c) removing the solvents at temperatures below the melting temperature of the scopolamine base.

## Revendications

1. Système thérapeutique transdermique qui contient de la scopolamine base à titre de substance active, comprenant une couche dorsale flexible imperméable à la substance active, une couche faisant office de réservoir contenant la substance active, une membrane de réglage, une couche adhésive pour la fixation du système sur la peau, ainsi qu'une feuille de protection à retirer avant l'application, également imperméable à la substance active, **caractérisé en ce que**
- la couche faisant office de réservoir, ainsi que la couche adhésive sont constituées par un polymère de silicone auto-adhésif aminorésistant, à titre de polymère de base,
- la membrane de réglage est constituée par un copolymère d'éthylène-acétate de vinyle, et
- la scopolamine base est présente pour une part, au moins dans la couche faisant office de réservoir, sous forme cristalline.

2. Système thérapeutique transdermique selon la revendication 1, **caractérisé en ce que** la membrane de réglage présente une teneur en acétate de vinyle entre 4,5 et 19 % et possède une épaisseur entre 30 et 100 µm.

3. Système thérapeutique transdermique selon la revendication 1 ou 2, **caractérisé en ce que** la couche faisant office de réservoir présente une teneur en scopolamine base entre 1,0 et 2,5 mg (g/g), de préférence entre 1,5 et 2,0 mg.

4. Système thérapeutique transdermique selon la revendication 1 ou 2, **caractérisé en ce que** la couche adhésive présente une teneur en scopolamine base entre 0,2 et 1,0 mg (g/g), de préférence entre 0,2 et 0,7 mg.

5. Système thérapeutique transdermique selon une ou plusieurs des revendications 1 à 4, **caractérisé en ce que**, aussi bien la couche faisant office de réservoir que la couche adhésive, contiennent une substance favorisant le taux de perméation à travers la peau humaine, de préférence un acide gras ou un alcool gras.

6. Système thérapeutique transdermique selon la revendication 5, **caractérisé en ce que** le renforçateur de la perméation est l'acide oléique.

7. Système thérapeutique transdermique selon la revendication 5, **caractérisé en ce que** le renforçateur de la perméation est l'alcool oléylique.

8. Procédé pour la préparation d'un emplâtre contenant de la scopolamine selon les revendications 1 à 7, dans lequel le procédé de préparation pour les couches contenant la substance active comprend les étapes indiquées ci-après consistant à :
a) mettre la scopolamine base en suspension dans une solution de l'adhésif aminorésistant à base de silicone,
b) enduire une feuille appropriée à l'aide de la suspension,
c) éliminer le solvant à des températures inférieures à la température de fusion de la scopolamine base.
